# EUROPEAN PATENT SPECIFICATION

(11) **EP 4 040 442 B1**
(45) Date of publication and mention of the grant of the patent: **28.05.2025**
(21) Application number: 21156011.5
(22) Date of filing: 09.02.2021
(51) Int. Cl.: G16H 10/40, G01N 35/00

(54) **LABORATORY DATA MANAGEMENT SYSTEM**
LABORDATENVERWALTUNGSSYSTEM
SYSTÈME DE GESTION DE DONNÉES DE LABORATOIRE

(43) Date of publication of application: 10.08.2022
(73) Proprietor: F. Hoffmann-La Roche AG, 4070 Basel (CH); Roche Diagnostics GmbH, 68305 Mannheim (DE); Hitachi High-Tech Corporation, Minato-ku Tokyo 105-6409 (JP)
(72) Inventor: ALLWINN, Richard, 82377 Penzberg (DE); SEYFFERT, Sascha, 82377 Penzberg (DE); HUSER, Ronald, 6343 Rotkreuz (CH); BITZER, André, 6343 Rotkreuz (CH); SCHWEIGHAUSER, Stephan, 6343 Rotkreuz (CH); KOIZUMI, Nobuhiro, Tokyo 1056409 (JP); SEKI, Yoshihiro, Tokyo 1056409 (JP)
(74) Representative: Altmann Stößel Dick Patentanwälte PartG mbB

(56) References cited:
- WO-A1-2015/013688
- WO-A1-2016/040985
- US-A1- 2007 129 894
- US-A1- 2014 129 172

## Description

### Technical Field

The present disclosure is related to a multi-layer laboratory data management system comprising lab devices as data source.

### Background

In the healthcare industry, data management systems for the automated in vitro diagnostic laboratory provide solutions for centralized management and standardized testing procedures by lab devices such as analyzers or other instruments.

In particular, middleware solutions are known that allow users to centrally manage patient and instrument data across lab devices through a wired connection or through handheld or mobile wireless devices.

With increasing complexity of lab configurations, e.g. with increasing number of lab devices to be managed and/or data being processed, data management may become more challenging. Typical challenges include, dealing with different communication formats, especially when lab devices are different from each other, and possibly include lab devices from different manufacturers, possibly using different control software, different software versions, different data format and different sets of data. Other challenges include making efficient use of collected lab device data in order to e.g. improve lab workflow, troubleshooting, serviceability and maintenance. Other challenges include lab device scalability, e.g. when adding new lab devices and/or new functionalities and/or when updating existing lab devices at hardware and/or software level. Other challenges include customization, e.g. if additional or new data management functions are desired that require additional data and/or use of the same data in a different manner or different combination. Other challenges include data security, data privacy and data accessibility.

WO 2016040985A1 discloses an instrument management system having a user device to receive inputs from a user. The user device has a device processor for processing the received inputs and instrument data from the instruments, and a device display for displaying instrument information. An interface module is communicatively coupled with the user device and the instruments to convert instrument data in a primary format generated by an instrument processor to a secondary format for processing by the device processor. No instrument data is stored at the interface module for later access.

### Summary of the Invention

The present invention provides a laboratory data management system as defined by appended claim 1. Preferred embodiments of the invention are set out in the dependent claims.

### General Description

In view of the above background, a laboratory data management system is herein disclosed that is format independent, scalable and customizable, and that enables to improve lab workflow, troubleshooting, serviceability and maintenance thereby making a more efficient use of collected lab device data. Other advantages will become clear throughout the description.

In particular, the laboratory data management system of the present disclosure comprises a data source layer comprising at least one laboratory device as a data source, a data adapter layer comprising at least one data source agent configured to obtain data from the at least one data source and to convert the data from a data source format to a data-source independent format, a consumer layer configured for installation and/or execution of consumer application software, and a data management layer between the data adapter layer and the consumer layer. The data management layer comprises a data storage configured to store data converted by the at least one data source agent and at least one data manager programmed to execute instructions from the consumer application software, which when executed cause the at least one data manager to select application-specific data in the data storage and to make them accessible to a consumer via the consumer application software in a consumer format.

A "laboratory device" or "lab device" for short can be any stand-alone apparatus or module within a larger connected system typically involved in sample processing for in-vitro-diagnostics. "Sample processing" means either detection, e.g. qualitative and/or quantitative evaluation of samples for diagnostic purpose, and/or sorting and/or preparation of samples before detection, or storing and/or disposal of samples after detection. In particular, a lab device may be related to analytical and/or to pre-analytical and/or to post-analytical sample processing steps. Lab devices may be connected to each other and depend at least in part on each other, e.g. each carrying out a dedicated task of a sample processing workflow, which may be a prerequisite before proceeding to the next lab device. Alternatively, lab devices may work independently from each other, e.g. each carrying out a separate task, e.g. a different type of analysis on the same sample or different sample.

An "analytical lab device" is either a stand-alone apparatus or module within a larger connected system configured for the detection, e.g. qualitative and/or quantitative evaluation of samples for diagnostic purpose. It may comprise a detection system and be configured to execute a workflow that is optimized for certain types of analysis. Examples of such analytical lab device are clinical chemistry analyzers, coagulation chemistry analyzers, immunochemistry analyzers, urine analyzers, hematology analyzers, molecular diagnostics analyzers, mass spectrometry analyzers, and the like (the list is not exhaustive) used to detect analytes in a sample or other sample parameters, typically as a result of chemical or biological reactions involving treating of samples with reagents. An analytical lab device can comprise functional units such as liquid handling units for pipetting and/or pumping and/or mixing of samples and/or reagents and/or system fluids, and also functional units for sorting, storing, transporting, identifying, separating, detecting. The analytical lab device may comprise a reagent holding unit for holding reagents to perform the assays. Reagents may be arranged for example in the form of containers or cassettes containing individual reagents or group of reagents, placed in appropriate receptacles or positions within a storage compartment or conveyor, e.g. a rotor. It may comprise a reaction vessel or cuvette feeding unit. In particular, it may comprise one or more liquid processing units, such as a pipetting unit, to deliver samples and/or reagents to the reaction vessels. The pipetting unit may comprise a reusable washable needle, e.g. a steel needle, or disposable pipette tips. The lab device may further comprise one or more mixing units, comprising e.g. a shaker to shake a cuvette comprising a liquid or a mixing paddle or stirrer to mix liquids in a cuvette or reagent container.

A "pre-analytical lab device" can be either a stand-alone apparatus or module within a larger connected system typically configured for sorting and/or checking sample quality and/or preparation of samples before being processed by an analytical lab device, possibly including process steps like reformatting, aliquoting and the like. It may comprise for example one or more of the following: a resorting unit to sort samples according to type of analysis and/or priority of analysis, a centrifuge for centrifuging sample tubes, an aliquoting unit where a pipetting unit is used to aliquot samples from sample tubes, a thermal treatment unit to subject the sample to a certain temperature, a separation unit to separate sample components, and the like.

A "post-analytical lab device" is either a stand-alone apparatus or module within a larger connected system typically configured for storing and/or disposal of samples after being processed by an analytical work cell. It may comprise for example a resorting or reformatting unit to resort sample tubes, e.g. to different storage racks, and/or a refrigerated compartment.

The term "lab device" may further include sample transportation devices or modules configured to transport samples between other lab devices, e.g. comprising conveyors either mechanical or based on other principles such as induction, to move samples, typically in sample tubes, on single-tube carriers or multi-tube carriers such as racks.

The term "lab device" may further include a robotic device programmed to interact with one or more other lab devices, e.g. to supply consumables to an analytical lab device or to perform maintenance tasks, in an automated manner.

Lab devices may have different configurations according to the need and/or according to the desired laboratory workflow. Additional configurations may be obtained by coupling a plurality of apparatuses and/or modules together, where each module may have a dedicated function. Thus each laboratory may be configured in a different manner according to user requirements and needs, e.g. in terms of sample processing throughput, type of analysis, type of samples and the like.

The term "data source layer" as used herein refers to a group of laboratory data producers whose produced data may be object of data management by the laboratory management system. In particular, the data source layer comprises at least a lab device as data source producing lab device data, and typically a plurality of lab devices. The data source layer may comprise also a middleware layer as data source that collects and manages data from one or more lab devices, e.g. a subgroup of lab devices, besides having possible other functions such as distributing data and instructions, e.g. analytical test orders, to one or more lab devices.

The term "lab device data" may refer to any data produced by a lab device, including but not limited to any one or more of events occurred during laboratory device use or maintenance, event-triggered alerts or messages, use and/or consumption data, laboratory device status or configuration data, laboratory device operational parameters, quality control (QC) data, lab device test result data. The term "operational parameter" may encompass any measurable parameter, or a property derivable from raw data, including any sort of distinguishable physical and/or chemical signal, that can be detected and quantified during operation of a lab device, such as but not limited to electrical current, voltage, electrical resistance, electrical capacitance, magnetic field, time, distance, size, shape, area, volume, height, velocity, position, temperature, pressure, viscosity, light intensity, wavelength, frequency, noise, and the like. The term "raw data" refers to data that has not been processed for use, but has the potential to become information by selective extraction, organization, and sometimes analysis and formatting for presentation. Once processed, the data can become an operational parameter. In particular, some operational parameters can be used to determine the operational status, e.g. performance status of a lab device, of individual modules and even individual functional (operational) components of a lab device. "Use data" once processed may include data analytics, e.g. statistical data with respect to effective use, throughput, downtime, frequency of particular events, statistical or actual workload, and the like. "Consumption data" once processed may refer to statistical or actual consumption of any consumables like reagents, system fluids, vessels, pipetting tips and the like.

The term "operation" or "operational" includes any time during which the lab device is occupied at processing samples or during which it is occupied with a quality or maintenance procedure or during which it is in an idle or standby status as long as data generation and collection can take place.

The term "data adapter layer" as used herein refers to a hardware and software layer whose main function is that of a protocol translator, by converting non-uniform data to uniform data for further processing by the data management layer. In particular, the data adapter layer comprises at least one data source agent, that is a logic controller, configured to obtain data from the at least one data source and to convert the data from a data-source format to a data-source independent format, e.g. to a proprietary format or standard format. Typically, the data adapter layer comprises a data source agent for each data source, e.g. a lab device agent for each lab device.

The data-source formats used in the lab device industry, although typically based on standards like the Health Level Seven or HL7, may be customized in a proprietary way that differ between different lab device manufacturers and even between different lab devices of the same lab manufacturer. In particular, different lab devices may be possibly using different control software, different software versions, different data format and different sets of data, and different communications protocols. It is thus advantageous for a consumer to have data from different data sources centrally converted from possible different data-source formats into a data-source independent format, so that they can be combined, compared and/or processed in the same manner.

According to an embodiment, the at least one data source agent is programmed to receive data as direct messages from the at least one data source or to query the at least one data source about specific data either in response to a particular one or more data-source events or at regular time intervals and to determine data changes between queries.

The term "consumer layer" as used herein refers to a hardware and software layer providing a user interface that enables a user to interact with the laboratory data management system. The user interface may be any sort of user interface like graphical user interface, command line interface, menu-driven user interface, touch user interface, voice user interface, form-based user interface, virtual reality user interface and can include the use of a display screen, a keyboard, a mouse, a computing device such as a desktop, laptop, tablet, smartphone or wearable device like a smartwatch or virtual reality devices, e.g. glasses configured to facilitate and enhance user experience. In particular, the consumer layer may comprise at least one and typically a plurality of consumer devices as interface device configured for the installation and/or the execution of consumer application software. According to an embodiment, the at least one consumer device is a mobile hand-held device such as a tablet device.

According to an embodiment, the consumer application software is downloadable from a cloud and installed onto the at least one consumer device.

According to an embodiment, the consumer application software is a cloud-based progressive web application (PWA) executable via the at least one consumer device.

In general, however, any form of native application software, locally or remotely executable, may be used.

A "data management layer" is a hardware and software layer between the data adapter layer and the consumer layer comprising a data storage configured to store data converted by the at least one data source agent and at least one data manager programmed to execute instructions from the consumer application software, which when executed cause the at least one data manager to select application-specific data in the data storage and to make them accessible to a consumer via the consumer application software in a consumer format.

According to an embodiment, the data management layer comprises a gatekeeper configured for filtering and validating data converted by the at least one data source agent.

The term "data storage" refers to any suitable memory device or medium such as a non-transitory computer readable medium or non-transitory computer readable memory and may be configured as a nonvolatile computer readable medium. The memory may comprise RAM, ROM, flash memories, hard drives, or any device capable of storing converted data in a way that can be accessed and eventually elaborated by the at least one data manager. The data storage may include other data in the form of machine readable instructions, e.g. logic or algorithm(s) written in a programming language such as, for example, machine language that may be directly executed by the at least one data manager, or assembly language, object-oriented programming (OOP), scripting languages, microcode, etc., that may be compiled or assembled into machine readable instructions and stored on the memory. Alternatively, the machine readable instructions may be written in a hardware description language (HDL), such as logic implemented via either a field-programmable gate array (FPGA) configuration or an application-specific integrated circuit (ASIC), or their equivalents. The data storage may be local or remote or distributed, in part local and in part remote, e.g. including a cloud storage.

The term "data manager" as used herein may refer to any physical or virtual processing device and in particular a programmable logic controller running a computer-readable program provided with instructions to perform operations in accordance with an operation plan. This may include a processor, a controller, a central processing unit (CPU), a microprocessor, a microcontroller, a reduced instruction circuit (RISC), an application-specific integrated circuit (ASIC), a logic circuit, or any other circuit or processor configured to execute one or more of the functions/methods described herein. In particular, there might be a plurality of data managers, e.g. a data manager for each application, e.g. a plurality of application-specific integrated circuits.

The one or more data managers may be integrated into the same hardware device as the data storage or be embodied at least in part as separate logic entities in communication with the data storage via a direct connection, wired or wireless, or indirectly over a communications network, wired or wireless, such as a wide area network, e.g., the Internet or a Health Care Provider's local area network or intranet, via a network interface device. In particular, the data manager and/or the data storage might be integral with a data management unit, e.g., implemented on a computing device such as a desktop computer, a laptop, a smartphone, a tablet, etc., may be comprised by a server computer and/or be distributed/shared across/between a plurality of devices. Moreover, the data management layer can include remote devices, servers and cloud-based elements that communicate via wires or wirelessly (e.g., infrared, cellular, Bluetooth^{®}), or a remote PC/server or a cloud-based system.

The term "remote system" or "server" as used herein encompasses any physical machine or virtual machine having a physical or virtual processor, capable of receiving; processing and sending data. A server can run on any computer including dedicated computers, which individually are also often referred to as "the server" or shared resources such as virtual servers. In many cases, a computer can provide several services and have several servers running. Therefore, the term server shall encompass any computerized device that shares a resource with one or more client processes. Furthermore, the terms "remote system" or "server" encompasses a data transmission and processing system distributed over a data network (such as a cloud environment).

The term "communication network" as used herein encompasses any type of wireless network, such as a WiFi^{™}, GSM^{™}, UMTS, LTE, 5G or other wireless digital network or a cable based network, such as Ethernet^{™} or the like. In particular, the communication network can implement the Internet protocol (IP). For example, the communication network comprises a combination of cable-based and wireless networks.

The at least one data source agent may collect and convert data and the data storage may store converted data independently from application software available to a consumer. However only a part of the stored data may be made accessible to the consumer, as a service, via consumer application software that is data-specific. In particular, a common lab data storage may be used for storing a record, e.g. in chronologic order, of any collected/converted data from any lab device, that can be accessed at a later time by adding new data/application-specific services.

According to an embodiment, the consumer application software and the application-specific data made accessible by the consumer application software are subscription-based.

According to an embodiment, the application-specific data comprise any one or more of events occurred during laboratory device use or maintenance, event-triggered alerts or messages, use and/or consumption data, laboratory device status or configuration data, laboratory device operational parameters, QC data, laboratory device test result data.

According to an embodiment, the consumer format comprises a report or display of application-specific data as such and/or after being elaborated into statistical, analytical or predictive data, e.g. by the at least one data manager.

According to an embodiment, the at least one data manager or the consumer application software includes a comparison function for comparing the application-specific data with predetermined reference or threshold values and an alert function or action trigger function in response to the application-specific data deviating from the reference or threshold values. According to an embodiment, the data source layer comprises a plurality of laboratory devices as data sources, including at least one analytical device and at least one robotic device configured to interact with the at least one analytical device.

According to an embodiment, the at least one data manager is programmed to process data from the at least one analytical device and responsive to the processed data to instruct the at least one robotic device directly or via the respective data source agent to interact with the at least one analytical device. The interaction, may include for example supplying consumables such as reagent containers, system fluid containers, pipetting tips, reaction vessels, and the like to the at least one analytical device, responsive to the processed data indicating a shortage of any of such consumables. The interaction may include also performing maintenance tasks, in an automated manner, such as e.g. cleaning or replacing parts, monitoring and reporting. The interaction may include also automatically intervening in case of emergency, e.g. by turning a lab device off or resolving a jam or the like responsive to alarms or initiating QC or other maintenance procedures based on trigger events. The robotic device may be for example configured as a humanoid robot capable of moving in a laboratory to and between other lab devices in order to physically interact with them, e.g. by gripping tools combined with other sensory tools and a logic processing function.

According to the claimed invention, the data adapter layer and the data management layer, at least in part, are arranged in a laboratory gateway connected to the data-source layer and to the consumer layer. The data storage may be entirely comprised in the gateway or distributed between the gateway and a separate device, include a remote location, e.g. a remote server or cloud. In further, non-claimed embodiments, the data storage may be however also entirely remote and connected to the gateway. According to an embodiment, the gateway is scalable by locally or remotely adding/removing/updating data source agents and/or data managers and/or services, e.g. as plug-in components and/or as local or remote system update.

According to an embodiment, adding new services comprises adding new data managers a priori, e.g. when new services become available by the service provider, e.g. as part of a system update, which can then be activated at any time by installing and/or subscribing to new consumer application software that communicates with the at least one previously added data manager thereby granting the consumer access to additional application-specific data in the data storage.

An advantage of such a gateway solution is to have a central data management system that enables reduction of dependency on operating system / software version of both the lab device(s) and the consumer device(s), thus reducing management effort of such devices and their maintenance costs, while enabling scalability (centrally managing data from additional lab devices added later and/or centrally adding more services at a later time) and interoperability. Also, having a common lab data storage, e.g. for storing a chronologic record of collected/converted data from a plurality of lab devices, e.g. event-based data, that can be accessed also at a later time by adding new services / new application software specific to a set of the stored data at later times, is particularly advantageous. Another advantage is the possibility to provide a safer cloud connectivity for remote access/ serviceability, remote storage or for cloud based services, as well as higher IT security, in terms of cyber security and data protection/privacy. Another advantage of having a central library of data in each laboratory, all converted into the same data-source independent format, is the possibility to interconnect different laboratories and/or to use data from different laboratory for interlaboratory comparison and improvement of lab configuration, troubleshooting, serviceability and maintenance.

Other and further objects, features and advantages will appear from the following description of exemplary embodiments and accompanying drawings, which serve to explain the principles more in detail.

### Brief Description of the Drawings

FIG. 1 shows schematically a multi-layer laboratory data management system and its main components according to the present disclosure.
FIG. 2 shows further aspects of the embodiment of FIG. 1 related to system scalability.
FIG. 3 shows further aspects of the embodiment of FIG. 1-2 involving the use of a robotic device.
FIG. 4 shows further components of the laboratory data management system of FIG. 1-3.

Skilled artisans appreciate that elements in the figures are illustrated for simplicity and clarity and have not necessarily been drawn to scale. For example, the dimensions of some of the elements in the figures may be exaggerated relative to other elements whereas other elements may have been left out or represented in a reduced number in order to enhance clarity and improve understanding of the embodiments of the present disclosure.

### Detailed Description

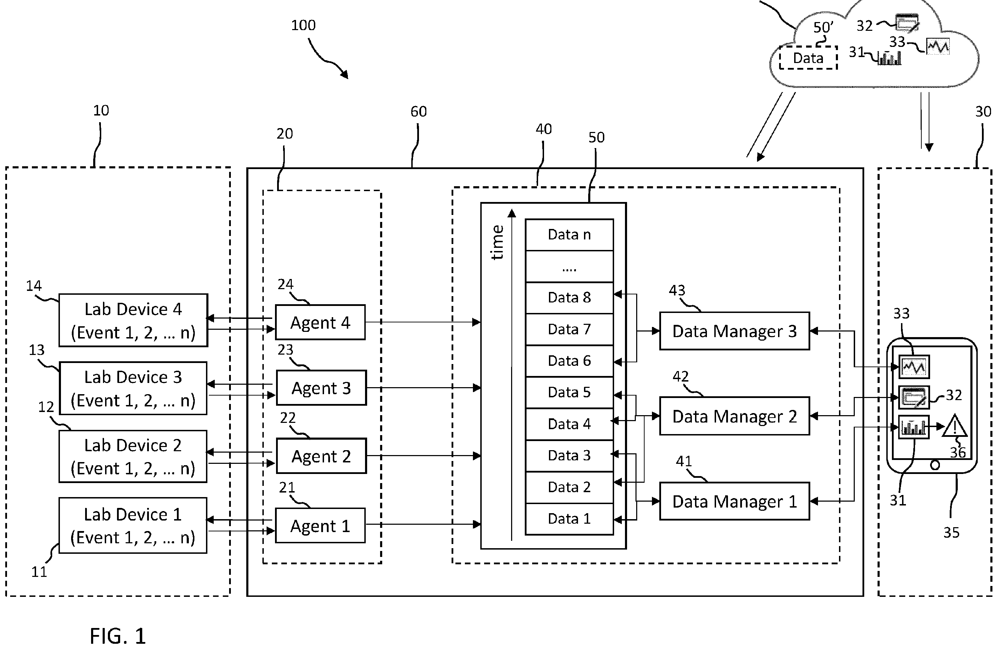

FIG. 1 shows schematically a laboratory data management system 100 comprising a data source layer 10 comprising a plurality of laboratory devices 11, 12, 13, 14 as data sources, a data adapter layer 20 comprising a plurality of data source agents 21, 22, 23, 24 configured to obtain data from the respective laboratory devices 11, 12, 13, 14 and to convert the data from a data source format to a data-source independent format, a consumer layer 30 configured for installation and/or execution of consumer application software 31, 32, 33, and a data management layer 40 between the data adapter layer 20 and the consumer layer 30. The data management layer 40 comprises a data storage 50 configured to store data 1-n converted by the data source agents 21, 22, 23, 24 and a plurality of data managers 41, 42, 43 programmed to execute instructions from respective consumer application software 31, 32, 33, which when executed cause the respective data manager 41, 42, 43 to select application-specific data out of data 1-n in the data storage 50 and to make them accessible to a consumer via the consumer application software 31, 32, 33 in a consumer format. In this schematic example, data 1, 3 are specific to consumer application software 31, data 2, 4, 5 are specific to consumer application software 32 and data 6, 8 are specific to consumer application software 33.

The data source agents 21, 22, 23, 24 are programmed to query the respective laboratory devices 11, 12, 13, 14 about specific data either in response to a particular one or more data-source events 1-n or at regular time intervals and to determine data changes between queries. The laboratory devices 11, 12, 13, 14 may be however configured to send data as direct messages to the respective data source agents 21, 22, 23, 24 for conversion. The data storage 50 is configured as a common data storage for storing any collected/converted data 1-n from any lab device 11, 12, 13, 14, e.g. in a chronologic order, from oldest to newest.

The consumer layer 30 comprises a consumer device 35 as interface device configured for the installation and/or the execution of the consumer application software 31, 32, 33. In this example, the consumer device 35 is a mobile hand-held device such as a tablet device. Also, although only one consumer device 35 is shown in FIG. 1, it is clear that the consumer layer may comprise any number of consumer devices of the same type or a combination of different types, synchronized or not between each other. In particular, some application software and/or access to one or more consumer devices may be consumer specific and/or may require a particular account or user authentication.

According to an embodiment, at least part of the consumer application software, e.g. consumer application software 32 and the respective application-specific data 2, 4, 5 out of the stored data 1-n that are made accessible to the consumer by the consumer application software 32 may require a subscription.

The consumer application software 31, 32, 33 may be downloadable from a cloud 70 and installed onto one or more consumer devices 35. Alternatively or in combination, the consumer application software 31, 32, 33 may be in the form of cloud-based progressive web application (PWA) executable via the one or more consumer devices 35, or any other form of native application locally or remotely executable.

The application-specific data may relate for example to any one or more of events 1-n occurred during use or maintenance of the laboratory devices 11, 12, 13, 14, including event-triggered alerts or messages, use and/or consumption data, laboratory device status or configuration data, laboratory device operational parameters, QC data, laboratory device test result data and the like.

The consumer format may comprise a report or display of application-specific data as such and/or after being elaborated into statistical, analytical or predictive data. As one example, consumption data of consumables, e.g. an overview of actual consumption of any consumables like reagents, system fluids, vessels, pipetting tips and the like by any one or more of the laboratory devices 11, 12, 13, 14, may be reported in any consumer format, such as graphical and/or text/numerical display. Also statistical consumption data, e.g. across different laboratory devices 11, 12, 13, 14 and/or in a time range, e.g. based on frequency and recurrence of particular analytical tests, may be reported. In addition, a consumption prediction may be provided based on such statistical data.

Any one or more data managers 41 or the consumer application software 31 may include a comparison function for comparing the application-specific data 1, 3 with predetermined reference or threshold values and an alert function or action trigger function 36 in response to the application-specific data 1, 3 deviating from the reference or threshold values, e.g. if any of the available consumables drop below a threshold value.

According to the embodiment shown in FIG. 1, the data adapter layer 20 and the data management layer 40 are arranged in a laboratory gateway 60 connected to the data-source layer 10 and to the consumer layer 30. The data storage 50 may be entirely comprised in the gateway or distributed between the gateway 60 and a separate device, include a remote location, e.g. comprise a remote data storage 50' on a remote server or the cloud 70. The data storage 50' may be entirely remote and connected to the gateway 60.

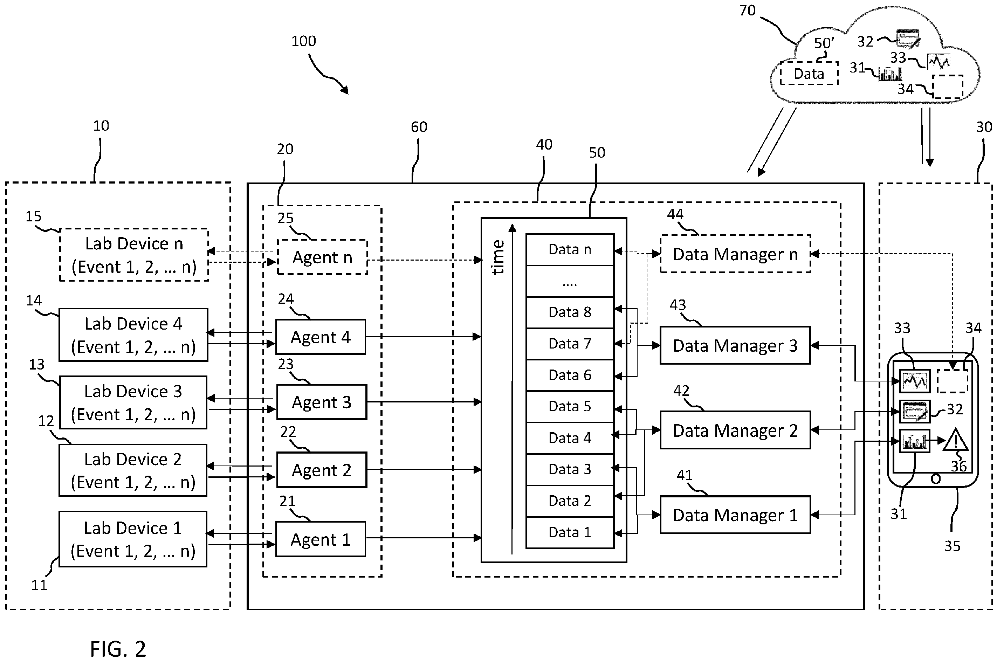

FIG. 2 shows further aspects of the embodiment of FIG. 1 and in particular an example of how the gateway 60 is scalable e.g. by adding a data source agent 25 in case of addition of laboratory device 15 to the data source layer 10 and/or by adding a data manager 44. This upgrade may be performed locally, e.g. by adding hardware components 25, 44 and/or executing a software update/installation for existing or new components 21, 22, 23, 24, 25, 41, 42, 43, 44 and could be performed also remotely in case of software update only. In particular, new services can be predisposed by adding e.g. new data managers 44 a priori, which can then be activated at any time by installing and/or subscribing to new consumer application software 34 that communicates with the previously added data manager 44 thereby granting the consumer access to additional (application-specific) data 7, n in the data storage 50.

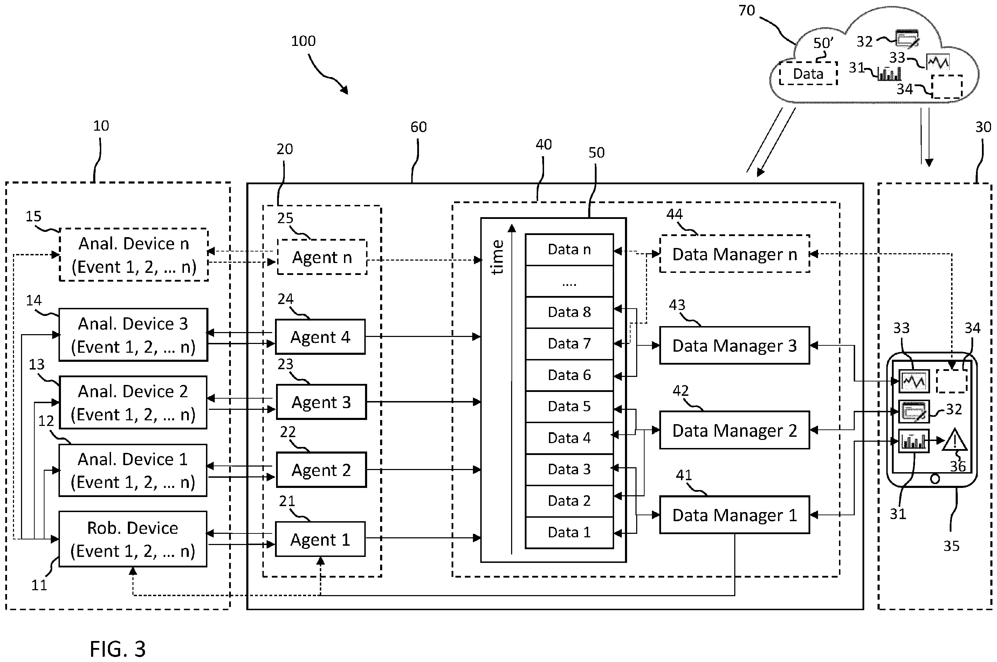

FIG. 3 shows further aspects of the embodiment of FIG. 1-2 where the plurality of laboratory devices 11, 12, 13, 14, 15 in the data source layer 10 include analytical devices 12, 13, 14, 15 and a robotic device 11 configured to interact with the analytical devices 12, 13, 14, 15. In particular, at least one data manager 41 is programmed to process data 1, 3 from any of the analytical devices 12, 13, 14, 15 and responsive to the processed data to instruct the robotic device 11 directly or via the respective data source agent 21 to interact with the one or more analytical devices 12, 13, 14, 15 which triggered a response. This process may occur in the background in a completely automated manner or may at least partly involve or at least inform a consumer, e.g. via the alert function 36 of the consumer application software 31. Also, the consumer application software 31 may be used to program the robotic device and/or to set alert rules or thresholds and respective actions. The robotic device 11 may be e.g. programmed to supply consumables such as reagent containers, system fluid containers, pipetting tips, reaction vessels, and the like to the analytical device 12, 13, 14, 15, responsive to the processed data 1, 3 indicating a shortage of any of such consumables or to perform maintenance tasks, in an automated manner or after initiation via the consumer application software 31.

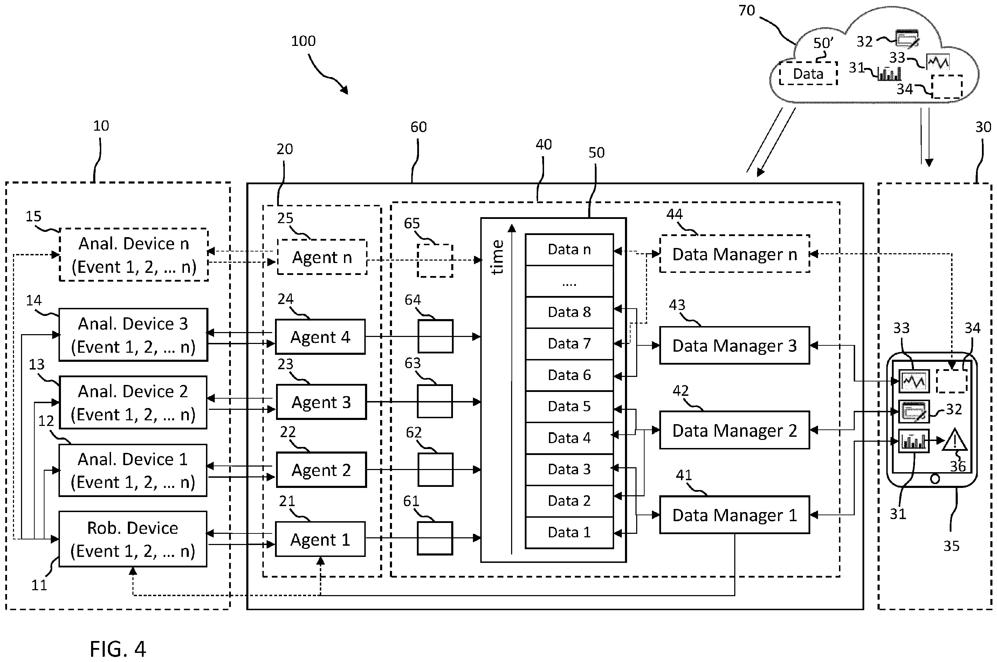

FIG. 4 shows further aspects of the embodiment of FIG. 1-3. In particular the data management layer 40 comprises gatekeepers 61, 62, 63, 64, 65 configured for filtering and validating data converted by the respective data source agents 21, 22, 23, 24, 25. This may be particularly important in an in-vitro diagnostic regulated and privacy-protected environment.

In the preceding specification, numerous specific details are set forth in order to provide a thorough understanding of the present disclosure. It will be apparent, however, to one having ordinary skill in the art that the specific detail need not be employed to practice the present teaching. In other instances, well-known materials or methods have not been described in detail in order to avoid obscuring the present disclosure.

Particularly, modifications and variations of the disclosed embodiments are certainly possible in light of the above description. It is therefore to be understood, that within the scope of the appended claims, the invention may be practiced otherwise than as specifically devised in the above examples.

Particularly, it is to be understood that the number of data sources, data source agents, data, data managers, consumer application software, consumer devices, gate keepers and of any other element shown is merely casual and provided as example only. Also the relationship between elements may be other than the one shown. For example there may be data source agents shared across different data sources or redundant data source agents communicating with the same data sources, although one data source agent for each data source is more typical. Likewise there may be fewer or more data managers with respect to the same set of data, e.g. configured to manage at least in part the same data, e.g. in different combinations for respective different services. Also, there may be fewer or more data managers with respect to the consumer application software, as one consumer application software may communicate with more than one data manager or vice versa. For example data elaborated by more data mangers may be combined and made accessible via the same consumer application software.

Reference throughout the preceding specification to "one embodiment", "an embodiment", "one example" or "an example", means that a particular feature, structure or characteristic described in connection with the embodiment or example is included in at least one embodiment. Thus, appearances of the phrases "in one embodiment", "in an embodiment", "one example" or "an example", in various places throughout this specification are not necessarily all referring to the same embodiment or example.

Furthermore, the particular features, structures, or characteristics may be combined in any suitable combinations and / or sub-combinations in one or more embodiments or examples.

## Claims

1. A laboratory data management system (100) comprising
- a data source layer (10) comprising at least one laboratory device (11, 12, 13, 14, 15) as a data source,
- a data adapter layer (20) comprising at least one data source agent (21, 22, 23, 24, 25) configured to obtain data from the at least one data source and to convert the data from a data source format to a data-source independent format,
- a consumer layer (30) configured for installation and/or execution of consumer application software (31, 32, 33, 34),
- a data management layer (40) between the data adapter layer (20) and the consumer layer (30) comprising
o a data storage (50) configured to store data (1-n) converted by the at least one data source agent (21, 22, 23, 24, 25),
o at least one data manager (41, 42, 43, 44) programmed to execute instructions from the consumer application software (31, 32, 33, 34), which when executed cause the at least one data manager (41, 42, 43, 44) to select application-specific data in the data storage (50) and to make them accessible to a consumer via the consumer application software (31, 32, 33, 34) in a consumer format,
wherein the data adapter layer (20) and the data management layer (40) are arranged in a laboratory gateway (60) connected to the data-source layer (10) and to the consumer layer (30).

2. The system (100) according to the preceding claim wherein the at least one data source agent (21, 22, 23, 24, 25) is programmed to receive data as direct messages from the at least one data source (11, 12, 13, 14, 15) or to query the at least one data source (11, 12, 13, 14, 15) about specific data either in response to a particular one or more data-source events (1-n) or at regular time intervals and to determine data changes between queries.

3. The system (100) according to claim 1 or 2 wherein the consumer layer (10) comprises at least one consumer device (35) as interface device configured for the installation and/or the execution of the consumer application software (31, 32, 33, 34).

4. The system (100) according to claim 3 wherein the consumer application software (31, 32, 33, 34) and the application-specific data made accessible by the consumer application software (31, 32, 33, 34) are subscription-based.

5. The system (100) according to claim 3 or 4 wherein the consumer application software (31, 32, 33, 34) is downloadable from a cloud (70) and installed onto the at least one consumer device (35) or wherein the consumer application software (31, 32, 33, 34) is a cloud-based progressive web application (PWA) executable via the at least one consumer device (35).

6. The system (100) according to any of the preceding claims wherein the application-specific data (1-n) comprise any one or more of events (1-n) occurred during laboratory device use or maintenance, event-triggered alerts or messages, use and/or consumption data, laboratory device status or configuration data, laboratory device operational parameters, QC data, laboratory device test result data.

7. The system (100) according to any of the preceding claims wherein the consumer format comprises a report or display of application-specific data as such and/or after being elaborated into statistical, analytical or predictive data.

8. The system (100) according to claim 7 wherein the at least one data manager (41) or the consumer application software (31) includes a comparison function for comparing the application-specific data (1, 3) with predetermined reference or threshold values and an alert function or action trigger function (36) in response to the application-specific data (1, 3) deviating from the reference or threshold values.

9. The system (100) according to any of the preceding claims wherein the data source layer (10) comprises a plurality of laboratory devices (11, 12, 13, 14, 15) as data sources, including at least one analytical device (12, 13, 14, 15) and at least one robotic device (11) configured to interact with the at least one analytical device (12, 13, 14, 15).

10. The system (100) according to claim 9 wherein the at least one data manager (41) is programmed to process data from the at least one analytical device (12, 13, 14, 15) and responsive to the processed data to instruct the at least one robotic device (11) directly or via the respective data source agent (21) to interact with the at least one analytical device (12, 13, 14, 15).

11. The system (100) according to any one of the preceding claims wherein the gateway (60) is scalable by locally or remotely adding/removing/updating data source agents (25) and/or data managers (44) and/or services.

12. The system according to claim 11 wherein adding new services comprises adding at least one new data manager (44) a priori, which can then be activated at any time by installing and/or subscribing to new consumer application software (34) that communicates with the at least one previously added data manager (44) thereby granting the consumer access to additional application-specific data (7, n) in the data storage (50).

13. The system (100) according to any of the claims 3 to 12 wherein the at least one consumer device (35) is a mobile hand-held device.

14. The system (100) according to any of the preceding claims wherein the data management layer (40) comprises one or more gatekeepers (61, 62, 63, 64, 65) configured for filtering and validating data converted by the at least one data source agent (21, 22, 23, 24, 25).

## Patentansprüche

1. Labordatenverwaltungssystem (100), umfassend
- eine Datenquellenebene (10), die mindestens eine Laborvorrichtung (11, 12, 13, 14, 15) als eine Datenquelle umfasst,
- eine Datenanpassebene (20), die mindestens einen Datenquellenagenten (21, 22, 23, 24, 25) umfasst, der dafür ausgebildet ist, Daten aus der mindestens einen Datenquelle zu erhalten und die Daten aus einem Datenquellenformat in ein Datenquellen-unabhängiges Format umzuwandeln,
- eine Verbraucherebene (30), die dafür ausgebildet ist, Verbraucheranwendungssoftware (31, 32, 33, 34) zu installieren und/oder auszuführen,
- eine Datenverwaltungsebene (40) zwischen der Datenanpassebene (20) und der Verbraucherebene (30), umfassend
∘ einen Datenspeicher (50), der dafür ausgebildet ist, Daten (1-n) zu speichern, die von dem mindestens einen Datenquellenagenten (21, 22, 23, 24, 25) umgewandelt wurden,
o mindestens einen Datenverwalter (41, 42, 43, 44), der dafür programmiert ist, Anweisungen von der Verbraucheranwendungssoftware (31, 32, 33, 34) auszuführen, die beim Ausführen den mindestens einen Datenverwalter (41, 42, 43, 44) veranlassen, anwendungsspezifische Daten in dem Datenspeicher (50) auszuwählen und diese über die Verbraucheranwendungssoftware (31, 32, 33, 34) einem Verbraucher in einem Verbraucherformat zugänglich zu machen,
wobei die Datenanpassebene (20) und die Datenverwaltungsebene (40) in einem Labor-Gateway (60) angeordnet sind, der mit der Datenquellenebene (10) und der Verbraucherebene (30) verbunden ist.

2. System (100) nach dem vorstehenden Anspruch, wobei der mindestens eine Datenquellenagent (21, 22, 23, 24, 25) dafür programmiert ist, Daten als direkte Nachrichten aus der mindestens einen Datenquelle (11, 12, 13, 14, 15) zu empfangen, oder die mindestens eine Datenquelle (11, 12, 13, 14, 15) bezüglich spezifischer Daten entweder als Reaktion auf ein bestimmtes Datenquellenereignis oder mehrere bestimmte Datenquellenereignisse (1-n) oder in regelmäßigen Zeitintervallen abzufragen und Datenänderungen zwischen Abfragen zu bestimmen.

3. System (100) nach Anspruch 1 oder 2, wobei die Verbraucherebene (10) mindestens eine Verbrauchervorrichtung (35) als Schnittstellenvorrichtung umfasst, die dafür ausgebildet ist, die Verbraucheranwendungssoftware (31, 32, 33, 34) zu installieren und/oder auszuführen.

4. System (100) nach Anspruch 3, wobei die Verbraucheranwendungssoftware (31, 32, 33, 34) und die anwendungsspezifischen Daten, die von der Verbraucheranwendungssoftware (31, 32, 33, 34) zugänglich gemacht werden, abonnementbasiert sind.

5. System (100) nach Anspruch 3 oder 4, wobei die Verbraucheranwendungssoftware (31, 32, 33, 34) aus einer Cloud (70) heruntergeladen werden kann und auf der mindestens einen Verbrauchervorrichtung (35) installiert ist oder wobei die Verbraucheranwendungssoftware (31, 32, 33, 34) eine cloudbasierte progressive Web-Anwendung (PWA) ist, die über die mindestens eine Verbrauchervorrichtung (35) ausführbar ist.

6. System (100) nach einem der vorstehenden Ansprüche, wobei die anwendungsspezifischen Daten (1-n) eines oder mehreres von Ereignissen (1-n), die während der Verwendung oder Wartung der Laborvorrichtung auftreten, durch ein Ereignis ausgelöste Alarme oder Nachrichten, Nutzungs- und/oder Verbrauchsdaten, Laborvorrichtungsstatus- oder -konfigurationsdaten, Laborvorrichtungsbetriebsparameter, QC-Daten, Laborvorrichtungstestergebnisdaten umfassen.

7. System (100) nach einem der vorstehenden Ansprüche, wobei das Verbraucherformat einen Bericht oder eine Anzeige von anwendungsspezifischen Daten als solche und/oder nachdem sie zu statistischen, analytischen oder prognostischen Daten verarbeitet worden sind, umfasst.

8. System (100) nach Anspruch 7, wobei der mindestens eine Datenverwalter (41) oder die Verbraucheranwendungssoftware (31) eine Vergleichsfunktion zum Vergleichen der anwendungsspezifischen Daten (1, 3) mit vorbestimmten Referenz- oder Schwellenwerten und eine Alarmfunktion oder Handlungsauslösefunktion (36) als Reaktion auf die anwendungsspezifischen Daten (1, 3), die von den Referenz- oder Schwellenwerten abweichen, einschließt.

9. System (100) nach einem der vorstehenden Ansprüche, wobei die Datenquellenebene (10) eine Vielzahl von Laborvorrichtungen (11, 12, 13, 14, 15) als Datenquellen umfasst, die mindestens eine Analysevorrichtung (12, 13, 14, 15) und mindestens eine Robotervorrichtung (11), die dafür ausgebildet ist, mit der mindestens einen Analysevorrichtung (12, 13, 14, 15) zu interagieren, einschließen.

10. System (100) nach Anspruch 9, wobei der mindestens eine Datenverwalter (41) dafür programmiert ist, Daten aus der mindestens einen Analysevorrichtung (12, 13, 14, 15) zu verarbeiten und als Reaktion auf die verarbeiteten Daten die mindestens eine Robotervorrichtung (11) direkt oder über den jeweiligen Datenquellenagenten (21) anzuweisen, mit der mindestens einen Analysevorrichtung (12, 13, 14, 15) zu interagieren.

11. System (100) nach einem der vorstehenden Ansprüche, wobei der Gateway (60) durch lokales oder entferntes Zufügen/Entfernen/Aktualisieren von Datenquellenagenten (25) und/oder Datenverwaltern (44) und/oder Diensten skalierbar ist.

12. System nach Anspruch 11, wobei das Zufügen neuer Dienste das Zufügen mindestens eines neuen Datenverwalters (44) a priori umfasst, der dann jederzeit durch Installieren und/oder Abonnieren auf neuer Verbraucheranwendungssoftware (34) aktiviert werden kann, die mit dem mindestens einen früher zugefügten Datenverwalter (44) kommuniziert, wodurch der Verbraucherzugriff auf zusätzliche anwendungsspezifische Daten (7, n) in dem Datenspeicher (50) gewährt wird.

13. System (100) nach einem der Ansprüche 3 bis 12, wobei die mindestens eine Verbrauchervorrichtung (35) eine mobile tragbare Vorrichtung ist.

14. System (100) nach einem der vorstehenden Ansprüche, wobei die Datenverwaltungsebene (40) einen oder mehrere Pförtner (61, 62, 63, 64, 65) umfasst, der/die dafür ausgebildet ist/sind, Daten, die von dem mindestens einen Datenquellenagenten (21, 22, 23, 24, 25) umgewandelt wurden, zu filtern und zu validieren.

## Revendications

1. Système de gestion de données de laboratoire (100) comprenant
- une couche source de données (10) comprenant au moins un dispositif de laboratoire (11, 12, 13, 14, 15) en tant que source de données,
- une couche adaptateur de données (20) comprenant au moins un agent source de données (21, 22, 23, 24, 25) configuré pour obtenir des données de l'au moins une source de données et pour convertir les données d'un format source de données en un format source de données indépendant,
- une couche consommateur (30) configurée pour l'installation et/ou l'exécution d'un logiciel d'application consommateur (31, 32, 33, 34),
- une couche de gestion de données (40) entre la couche adaptateur de données (20) et la couche consommateur (30) comprenant
∘ un stockage de données (50) configuré pour stocker des données (1-n) converties par l'au moins un agent source de données (21, 22, 23, 24, 25),
o au moins un gestionnaire de données (41, 42, 43, 44) programmé pour exécuter des instructions du logiciel d'application consommateur (31, 32, 33, 34), qui lorsqu'elles sont exécutées amènent l'au moins un gestionnaire de données (41, 42, 43, 44) à sélectionner des données spécifiques à l'application dans le stockage de données (50) et à les rendre accessibles à un consommateur par l'intermédiaire du logiciel d'application consommateur (31, 32, 33, 34) dans un format consommateur,
dans lequel la couche adaptateur de données (20) et la couche de gestion de données (40) sont agencées dans une passerelle de laboratoire (60) connectée à la couche source de données (10) et à la couche consommateur (30).

2. Système (100) selon la revendication précédente dans lequel l'au moins un agent source de données (21, 22, 23, 24, 25) est programmé pour recevoir des données en tant que messages directs de l'au moins une source de données (11, 12, 13, 14, 15) ou pour interroger l'au moins une source de données (11, 12, 13, 14, 15) au sujet de données spécifiques soit en réponse à un ou plusieurs événements source de données (1-n) particuliers soit à intervalles de temps réguliers et pour déterminer des changements de données entre les requêtes.

3. Système (100) selon la revendication 1 ou 2 dans lequel la couche consommateur (10) comprend au moins un dispositif consommateur (35) en tant que dispositif interface configuré pour l'installation et/ou l'exécution du logiciel d'application consommateur (31, 32, 33, 34).

4. Système (100) selon la revendication 3 dans lequel le logiciel d'application consommateur (31, 32, 33, 34) et les données spécifiques à l'application rendues accessibles par le logiciel d'application consommateur (31, 32, 33, 34) sont basés sur un abonnement.

5. Système (100) selon la revendication 3 ou 4 dans lequel le logiciel d'application consommateur (31, 32, 33, 34) peut être téléchargé à partir d'un cloud (70) et installé sur l'au moins un dispositif consommateur (35) ou dans lequel le logiciel d'application consommateur (31, 32, 33, 34) est une application web progressive (PWA) basée sur le cloud pouvant être exécutée par l'intermédiaire de l'au moins un dispositif consommateur (35).

6. Système (100) selon l'une quelconque des revendications précédentes dans lequel les données spécifiques à l'application (1-n) comprennent un quelconque ou plusieurs des événements (1-n) survenus pendant l'utilisation ou la maintenance du dispositif de laboratoire, des alertes ou des messages déclenchés par un événement, des données d'utilisation et/ou de consommation, un statut ou des données de configuration du dispositif de laboratoire, des paramètres de fonctionnement du dispositif de laboratoire, des données de CQ, des données de résultats de test du dispositif de laboratoire.

7. Système (100) selon l'une quelconque des revendications précédentes dans lequel le format consommateur comprend un rapport ou un affichage de données spécifiques à l'application en tant que telles et/ou après avoir été élaborées en données statistiques, analytiques ou prédictives.

8. Système (100) selon la revendication 7 dans lequel l'au moins un gestionnaire de données (41) ou le logiciel d'application consommateur (31) comprend une fonction de comparaison pour comparer les données spécifiques à l'application (1, 3) à des valeurs de référence ou seuil prédéterminées et une fonction d'alerte ou une fonction de déclenchement d'action (36) en réponse aux données spécifiques à l'application (1, 3) s'écartant des valeurs de référence ou seuil.

9. Système (100) selon l'une quelconque des revendications précédentes dans lequel la couche sources de données (10) comprend une pluralité de dispositifs de laboratoire (11, 12, 13, 14, 15) en tant que sources de données, comprenant au moins un dispositif analytique (12, 13, 14, 15) et au moins un dispositif robotisé (11) configuré pour interagir avec l'au moins un dispositif analytique (12, 13, 14, 15).

10. Système (100) selon la revendication 9 dans lequel l'au moins un gestionnaire de données (41) est programmé pour traiter des données de l'au moins un dispositif analytique (12, 13, 14, 15) et en réponse aux données traitées pour donner l'instruction à l'au moins un dispositif robotisé (11) directement ou par l'intermédiaire de l'agent source de données (21) respectif d'interagir avec l'au moins un dispositif analytique (12, 13, 14, 15).

11. Système (100) selon l'une quelconque des revendications précédentes dans lequel la passerelle (60) peut être mise à l'échelle en ajoutant/retirant/mettant à jour localement ou à distance des agents sources de données (25) et/ou des gestionnaires de données (44) et/ou des services.

12. Système selon la revendication 11 dans lequel l'ajout de nouveaux services comprend l'ajout d'au moins un nouveau gestionnaire de données (44) a priori, qui peut ensuite être activé à n'importe quel moment en installant et/ou en s'abonnant à un nouveau logiciel d'application consommateur (34) qui communique avec l'au moins un gestionnaire de données (44) précédemment ajouté accordant ainsi au consommateur l'accès à des données supplémentaires spécifiques à l'application (7, n) dans le stockage de données (50).

13. Système (100) selon l'une quelconque des revendications 3 à 12 dans lequel l'au moins un dispositif consommateur (35) est un dispositif mobile portatif.

14. Système (100) selon l'une quelconque des revendications précédentes dans lequel la couche de gestion de données (40) comprend un ou plusieurs contrôleurs d'entrée (61, 62, 63, 64, 65) configurés pour filtrer et valider des données converties par l'au moins un agent source de données (21, 22, 23, 24, 25).
